# EUROPEAN PATENT APPLICATION

(11) **EP 1 983 026 A1**
(43) Date of publication of application: **22.10.2008**
(21) Application number: 07106385.3
(22) Date of filing: 18.04.2007
(51) Int. Cl.: C08L 23/00, C08L 73/02, D01F 6/46, D01F 8/06, D06M 16/00, A61K 8/72, C08L 23/36, C08F 8/32, A61K 8/43, D04H 1/00

(54) **Modified Polyolefin Surfaces**

(71) Applicant: The Procter and Gamble Company, Cincinnati, Ohio 45202 (US)
(72) Inventor: Casas-Sanchez, Jorge, 08820, El Prat de Llobregat (Barcelona) (ES); Herrlein, Matthias Kurt, 65719, Hofheim (DE); Collias, Dimitris Ioannis, Mason, OH 45040 (US); Mitchell, Michael Donovan, Cincinnati, OH 45251 (US); Wehmeier, Thomas Joseph, Cincinnati, OH 45233 (US)
(74) Representative: Borbach, Markus

(57) **Abstract**

Surface modifieable polyolefins are disclosed that allow for the covalent attachment of further chemical entities. The surface modification does not leach from the polyolefin. Example embodiments include the covalent bonding of antimicrobial chemical entities to the modified surfaces to provide antimicrobial polyolefin films and fibres for use in packaging and non-wovens.

## Description

### FIELD OF THE INVENTION

Polyolefin materials are well known in the art with a diverse range of applications. In particular polyolefin fibres have been widely used in the non-wovens industry in the manufacture of non-woven webs, fabrics and composites. Some applications are however limited by the inert and hydrophobic chemical properties of polyolefins. The invention relates to the formation of polyolefin materials that have a modified surface that allows for chemical modification of the surface properties.

### BACKGROUND OF THE INVENTION

Polyolefin polymers are widely used throughout the world for a variety of different processes and products. These include injection and blow molded articles, films, foams, mono filaments and nonwoven materials. This versatility in use comes from the polyolefin's good mechanical properties, chemical inertness and low cost. For many of these purposes the hydrophobic and chemically unreactive surface of the polyolefin material is a clear advantage. However, there are several applications where these characteristics actually limit the usefulness of the material.

For example of issues with hydrophobicity, the use of polyolefin fibres in nonwoven webs makes the resulting material strongly resistant to the take up of moisture. This means they are unsuitable to be used in absorbent articles to make diapers or wipes without further treatment to adjust their properties. Likewise the hydrophobic surface character of the polyolefin molded materials causes issues with the adhesion of paints to the surface.

Normal polyolefin fibres are unsuitable for use in paper making due to their insolubility in pulp mixtures. Surface wettable, highly hydrophilic polyolefin fibres have to be used in the paper industry to make high quality paper.

There are several techniques applied to making polyolefin fibres and materials more hydrophilic or more reactive to other chemicals. These include the use of monomers of different chemicals with the polyolefin monomers to produce polymers with different properties along their length, the use of block copolymers with the polyolefin monomers, post treatment of the polyolefin material with additives and the grafting of suitable hydrophilic agents to the surface of the polyolefins through radical chemical reactions.

US 6,696,373 discloses polyolefin fibres modified by the inclusion of long chain fatty acids to give hydrophilic fibres.

US 5993,840 discloses the use of PHMB with cellulosic non wovens in disposable articles such as nappies.

There is a need for polyolefins that can have their surface chemistry readily modified to whatever property is desired. There is a need for this to be carried out simply without complex and difficult post formation modification. It is also desirable to have a method that affects the surface of the polyolefin while leaving the core of the polyolefin unchanged to enable the beneficial structural properties associated with the material to remain.

### SUMMARY OF THE INVENTION

The invention describes the formation of a surface modified polyolefin with a non-leaching, reactive chemical functionality. The surface modified polyolefin comprising;
i) from 5 - 95% of a polyolefin; and
ii) from 5 - 95% of an amphiphilic polymer containing reactive chemical functionality
   where at least a portion of the reactive chemical functionality is available for chemical reaction at the surface of the polyolefin.

The invention describes a particular embodiment that consists of a multi layer fibre or film such that at least one layer is formed from the surface modified polyolefin. It is a further aspect of this invention to react the multilayer fibres or films with other molecules to induce other properties such antimicrobial activity or softness etc.

### BRIEF DESCRIPTION OF THE DRAWINGS

Figure 1 shows the IR spectrum of the PA-18 polymer.
Figure 2 shows the IR spectrum of PHMB.
Figure 3 shows the IR spectrum of the PHMB treated with surface modified film.
Figure 4 shows the O *1s* XPS spectra for the unmodified polypropylene control.
Figure 5 shows the O *1s* XPS spectra for the PA-18 modified film.
Figure 6 shows the O *1s* XPS spectra for the PHMB treated PA-18 modified film..
Figure 7 shows the C *1s* XPS spectra for pure polypropylene.
Figure 8 shows the C *1s* XPS spectra of a PA-18 modified film.
Figure 9 shows the C *1s* XPS spectra for the PHMB treated PA-18 modified film.

### DETAILED DESCRIPTION OF THE INVENTION

All percentages are by weight unless specifically stated otherwise.

As used in the present invention "non-leaching" means the group is attached to the surface and does not desorb from the polyolefin surface.

The term "polyolefin" as used in the present invention is defined as a polymer of simple olefin monomers. Non limiting examples include ethylene, propylene, butenes, isoprenes, and pentenes. Polyolefins consist only of carbon and hydrogen atoms and they are non-aromatic. Polyolefins are usually processed by extrusion, injection molding, blow molding, and rotational molding methods while thermoforming, alendaring, and compression molding are used also used but to a lesser degree.

The term "amphiphilic polymer" as used herein is to describe a polymer that has portions that are non-polar and portions which are polar. The two portions may be evenly distributed in regularly alternating sections along the length of the polymer or in a random arrangement of polar and non-polar sections.

The term "reactive chemical functionality" as used in the present invention is used to describe a functional group that is readily chemically reacted under standard conditions. The group does not comprise alkanes or unsubstituted aromatic groups.

The purpose of the invention is to produce a polyolefin surface that is readily susceptible to further chemical modification while maintaining as much as possible the key physical properties of the polyolefin.

Particular non-limiting examples of polyolefins useful for the present invention are polyethylene, and polypropylene.

The objective is achieved by the incorporation of an amphiphilic polymer into the surface of the polyolefin. The amphiphilic polymer is melt extruded with the polyolefin to generate polymers that are surface rich in the amphiphilic polymer. Without wishing to be bound by theory the applicants believe that some of the amphiphilic polymer is drawn to the polyolefin surface. The non-polar groups on the polymer have a favourable interaction with the olefin polymer chains while the polar groups are strongly repulsed by the olefin polymer chains. These interactions lead to the surface orientation of the amphiphilic polymer with the non polar portions interacting with the olefin polymer and the polar groups orientated away from the polyolefin at the surface to minimize the unfavourable interactions.

Non limiting examples of chemical functionality that could be described as non polar would be alkyl, alkenyl, alkynyl, halide and aryl groups, effectively any functional group that is made from predominantly only carbon and hydrogen atoms. Non limiting examples of chemical functionality that could be described as polar groups would be, amines, amides, carboxylic acids, carboxylic acid anhydrides, epoxies, ketones, aldehydes, heterocycles, nitrogen oxides, sulfides, sulfites, sulfates, phosphates, borates etc.

The amphiphilic polymer incorporates polar functionality that can be readily reacted with under standard chemical reactions and unreactive non-polar groups that are inactive to chemical reaction.

Amphiphilic polymers that are particularly interesting to the present invention are those that consist of a polar group containing a carboxylic acid or carboxylic acid anhydride functionality and a non polar group consisting of an alkane portion. The chain may be straight or branched. The alkane non polar group gives a strong interaction with the polyolefin and ensures a good mix. The acid or anhydride functionality gives hydrogen bonding ability which increases the hydrophilicity of the material and can be readily reacted to form stable covalent bonds with other chemical agents.

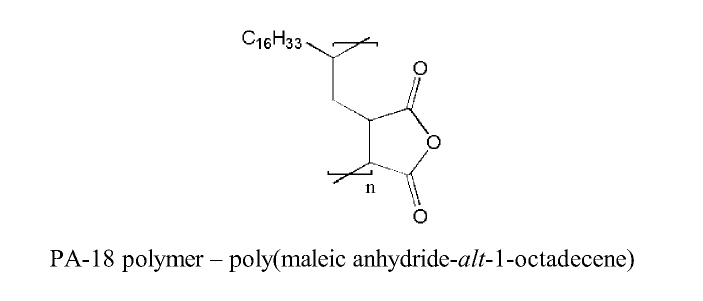

A particularly preferred amphiphilic polymer is PA-18 (poly(maleic anhydride-*alt*-1-octadecene)) which is a polyanhydride derived from the reactions of 1-octadecene and maleic anhydride in a 1:1 molar ratio and is commercially available from Chevron Philips Chemical (The Woodlands, TX). PA-18 has a repeat unit that has a C-18 alkane chain coupled to an acid anhydride. The C-18 group provides the non-polar, portion of the polymer. This portion readily interacts with the polyolefin core of the film or fibre. The acid anhydride provides the polar group on the polymer. The anhydride group is very labile and can readily undergo further chemical modification if desired. The anhydride may be hydrolyzed in the presence of base to give the bis carboxylic acid.

Other derivatives of this polymer with different carbon chain lengths could also be used. Carbon spacing chains between anhydride groups could be between 12 and 50 carbons long. The choice will depend on the properties desired in the finished film or fibre. Using a shorter chain length will give an increased coverage of polar groups on the surface of the polyolefin.

The surface modified polyolefins are produced by melt extruding a mixture of the olefin and the amphiphilic polymer. This can be achieved via any process known in the art. Due to the electronic interactions the amphiphilic polymer will be found in highest concentration at the surface of the olefin.

The amount of amphiphilic polymer that may be used in the preparation of the surface modified polymer is at least 1%, or alternatively at least 5%, or alternatively at least 10% or alternatively at least 20%, or alternatively at least 25%, or alternatively at least 30%, or alternatively at least 40%, or alternatively at least 50%, or alternatively at least 75%, or alternatively at least 90%, or alternatively at least 95% by weight of the polyolefin.

The modified polyolefin can be used on its own to form, for example, blow and injection molded products, fibres for the manufacture of non-wovens and films for packaging and sealing. The reactive chemical groups at the surface can then be reacted further to add other functions or properties.

The modified polyolefin polymer can also be used as a thin layer on an unmodified polyolefin surface to form laminate or bi-component products. This is particularly desirable as it reduces the quantity of the modified polymer required to enable the surface properties of polyolefin products to be altered. Blow molding in particular is a useful technique to create layered polyolefin materials. A bottle could be constructed predominantly from a polyolefin fibre and given a thin coating of the modified polyolefin fibre. The coating would then enable the attachment to the surface of other chemical groups, non limiting examples of which would be sensors, dyes or antimicrobial compounds, for example.

Melt extruding gives the ability to make multilayer (laminate) or bi-component fibres and multilayer films.

Fibres can be constructed in a core and sheath arrangement for example. The core layer would be constructed from a non modified polymer and the sheath layer of would contain the surface modified polyolefin fibre containing the amphiphilic polymer.

In a bi-component fibre, the "sheath" or surface layer will be formed from the surface modified polyolefin and make up at least 1%, or alternatively at least 5%, or alternatively at least 10%, or alternatively at least 20%, or alternatively at least 25%, or alternatively at least 33%, or alternatively at least 50%, or alternatively at least 75%, alternatively at least 90%, or alternatively at least 95% of the weight of the total fibre.

In a laminate film, the layer of surface modified polyolefin will consist of at least 0.5%, or alternatively at least 1%, or alternatively at least 5%, or alternatively at least 10%, or alternatively at least 20%, or alternatively at least 25%, or alternatively at least 33%, or alternatively at least 50%, or alternatively at least 75%, or alternatively at least 90%, or alternatively at least 95% of the weight of the total film.

The surface or sheath layer may also contain other optional ingredients as required.

### Modification of the reactive chemical functionality

The polar functionality of the amphiphilic groups at the surface of the film or fibre may be enough to give the desired properties (wetness, ability to accept a coating) in themselves, depending on the groups used. However the polar functionality may be reacted with further chemicals to give the required properties. This may be simply to carry out functional group transformations of the polar groups on the amphiphilic polymer to other more desired groups. Carboxylic acids reduced to alcohols, or reacted with amines to form amides, for example.

Alternatively the groups on the amphiphilic polymer may be used as attachment sites to covalently bind other chemical entities to the film or fibre. These chemical entities may be used to provide an additional property. Non limiting examples of chemical entities that may be added include antimicrobial entity, a photo absorbing compound, a sensor capable of reacting to temperature or pH for example, a conducting material, etc.

A particularly useful property would be the formation of antimicrobial polyolefin films and fibres. Thin polyolefin based films are used to package a wide range of commercially available products from foods and medicines to clothing and general consumer goods. For foods and medical items it would be especially advantageous to produce films that are resistant to microbial life.

Antimicrobial entities can take many forms. A non limiting list of examples of antimicrobial entities that could be reacted to a modified polyolefin include quaternised nitrogen containing species and groups containing silver or other metal atoms.

A particularly desirable antimicrobial agent is polyhexamethylene biguanide (PHMB). The polymer consists of a repeat unit of a highly basic biguanide group which remains in protonated form to about pH 10 and a hexane group and the polymer has an average of 10 - 12 repeat units. The advantages associated with PHMB include low skin irritancy, low eye toxicity, rapid kill, and stability and effectiveness over a wide pH range (2-11).

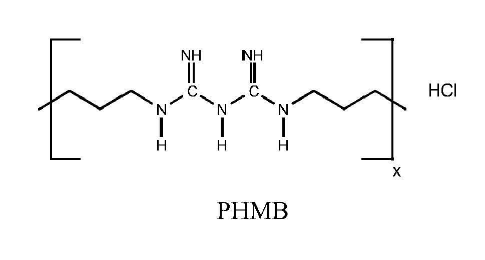

The PHMB can become tethered to the surface modified polyolefin when the reactive chemical functionality on the amphiphilic polymer is an acid anhydride or carboxylic acid. These groups readily react with the amine function of the PHMB to form very strong covalent bonds.

In an embodiment where the amphiphilic polymer is PA-18 the PHMB can be reacted under basic conditions to form a covalently bonded product.

The amount of PHMB that can be used in at least 0.001 %, or alternatively at least 0.01 %, or alternatively at least 0.1 %, or alternatively at least 0.5%, or alternatively at least 1 %, or alternatively at least 2%, or alternatively at least 5%, or alternatively at least 10%, or alternatively at least 20%, or alternatively at least 30% by weight of the modified polyolefin.

Other examples of antimicrobial compounds that can be used include quaternised PEI (polyethylene imine).

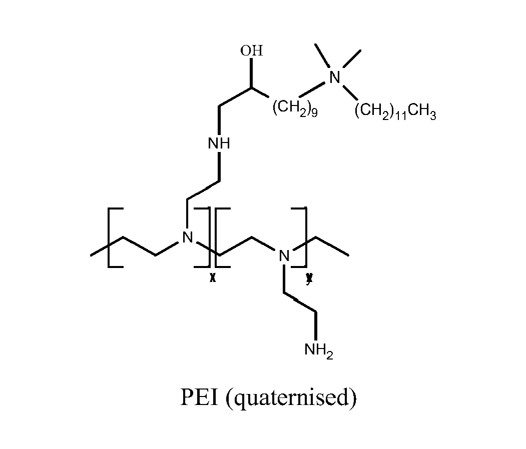

This technology particularly applies to non woven materials constructed from polyolefin fibres that are used to make absorbent articles. Wound dressings, diapers and wipes for example would all benefit from being inherently antimicrobial to prevent infection being caused through the use of contaminated materials. Through the reacting of antimicrobial agents to the surface polar groups of the modified polyolefin fibres these agents may become covalently bound to the groups. This provides excellent microbial control with a minimum amount of antimicrobial agent. With the groups tethered to the surface of the polyolefin, leaching and potential contamination of other surfaces is not possible.

One embodiment of this invention would be to use the modified fibres to form very mild antimicrobial wet wipes. While not limiting to any particular purpose, such wipes may be intended towards the cleaning of the body, particular the peri-anal area and/or external genital area of babies.

Wet wipes consist generally of a fibrous substrate impregnated with a lotion. The substrates are typically nonwoven and can be formed from synthetic and natural fibres or mixtures thereof. Example fibres include polypropylene, PET, cellulose, polyamides, nylon, rayon, lyocell, cotton, polyester, polyvinyl alcohols etc The substrates can be formed by any production process known in the art, non limiting examples including air laying and wet laying with bonding steps such as hydroentanglement, chemical and thermal bonding. Suitable substrates for wet wipes typically have a dry basis weight of between 30 grams per square metre (gsm) and 100 gsm, more precisely 40 gsm to 80 gsm and more precisely 45 gsm to 65 gsm.

Lotions suitable for wet wipes can be water based, or based on other solvents such as ethanol. Lotions may also take the form of emulsions or creams. Lotions can comprise many optional ingredients such as surfactants, emollients, scents, natural oils, thickeners, etc.

An example nonwoven substrate for wipes would comprise between 0% and 100% by weight of the modified polyolefin fibres, alternatively 20% to 80% by weight, or alternatively 40% to 60% by weight. The remaining weight may be unmodified polyolefin fibres or any other fibre type or mixture of fibres, synthetic or natural or combinations thereof. The modified fibres of the web being further treated with an anti microbial agent.

The antimicrobial treatment of the modified fibre can occur prior to the formation of the nonwoven substrate or post formation of the substrate.

Surface modified polyolefin fibre treated with PHMB has been demonstrated to be highly active against *E*. *coli.* Wipes made from such fibres will be able to utilize much lower quantities of preservative chemicals in their lotions while still remaining uncontaminated by microbial life. This is highly desirable feature as preservative chemicals are often harsh and can be deposited on the skin or other surfaces during use. With the PHMB bound to the surface of the wipes of the present invention, this situation can be avoided, ensuring a very mild product.

### Example 1 - Generating surface modified polyolefins

A blend of 75/25 polypropylene/PA-18 was supplied to a Haake Rheocord 90 melt extruder to melt and disperse the PA-18 into the polypropylene matrix. The resulting extrudate was then passed through a grinder to produce plastic chips of the size necessary to feed conventional fiber spinners or injection mold machines.

### Example 2 - Preparing bi-component fibres with a core of polypropylene and a sheath of surface modified polypropylene.

The blend chips of surface modified polypropylene generated in example 1 were then fed into a spinner capable of creating bi-component fibers. Fibres were then spun with a core of pure polypropylene with a sheath made from the 75/25 blend of polypropylene/PA-18 from Example 1. The ratio of core to sheath was 66 to 33 %. The fibres produced were identical in appearance from regular polypropylene fibres and can be substituted for regular polypropylene fibres for all uses known in the art. It will be obvious to the skilled person that the ratio of core to sheath can easily be varied according to need.

### Example 3 - Addition of antimicrobial agent to modified polypropylene surface

An aqueous solution of PHMB was added to a 500 mL resin kettle and the pH was adjusted to 10 with the addition of NaOH. The modified fibres from example 2 above were added to the kettle and the resulting solution was refluxed for 1 hour. After allowing the solution to cool the fibers were rinsed with deionised water and dried.

### Experimental Data

### Elemental Analyses

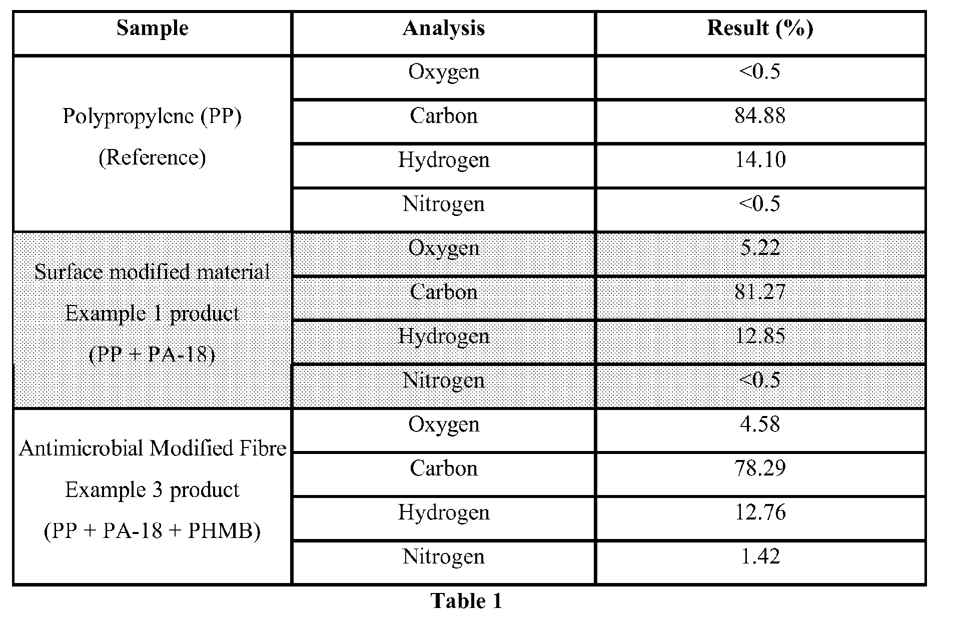

Elemental analysis was carried out on the samples to determine the atomic composition. From this data the extent of the modification to the polymer can be gauged. Elemental analysis of the oxygen content of the surface modified polypropylene product, Example 1, demonstrates that the ratio of polypropylene to PA-18 is approximately 60:40. From the elemental analysis of the nitrogen content of the PHMB modified bi-component fibre from Example 3, it is possible to determine that PHMB accounts for approximately 4% of the total weight, or approximately 10% of the surface modified sheath layer. The elemental analysis carried out on these samples demonstrates a much modified chemistry in comparison with polypropylene.

### IR Data

Infra-red spectroscopy is a powerful and sensitive method for determining the functional groups present in a composition. Changes in to these groups following chemical reaction are readily highlighted.

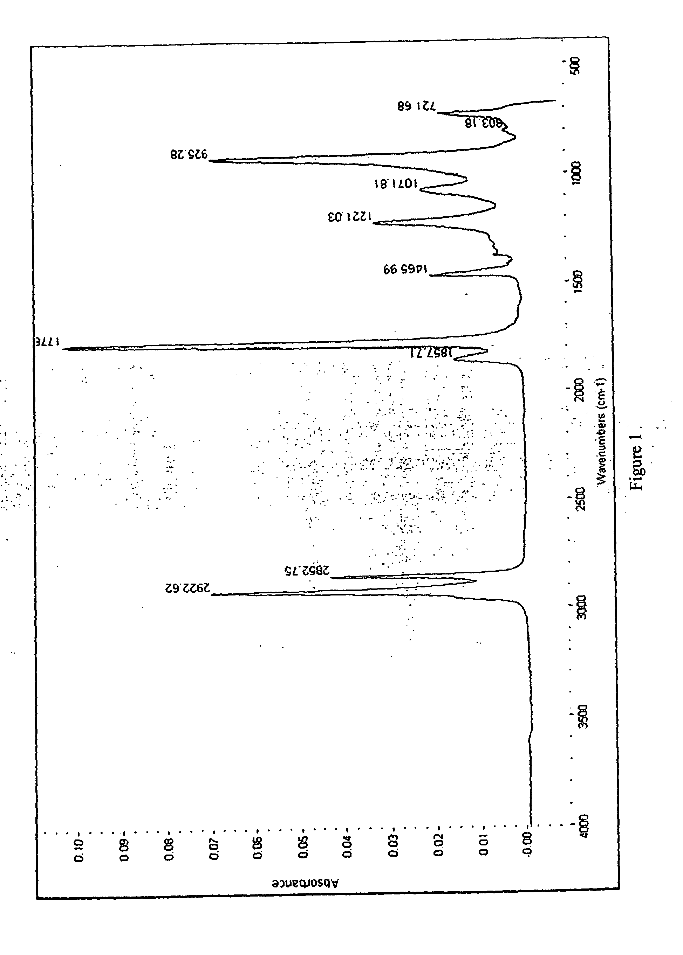

Figure 1 shows the IR spectrum of the PA-18 polymer. The two peaks for the anhydride functionality in the polymer can be seen at 1778 and 1857 cm⁻¹.

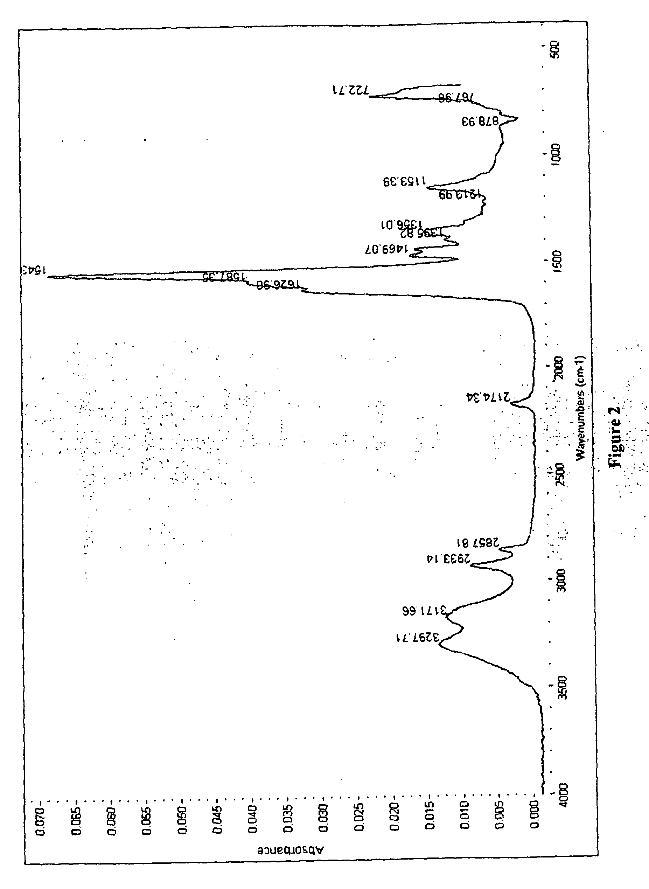

Figure 2 shows the IR spectrum of PHMB. The spectra reveals peaks at 3297 and 3171 cm⁻¹ that are representative for N-H bonds and a weak C=N peak at 2174 cm⁻¹.

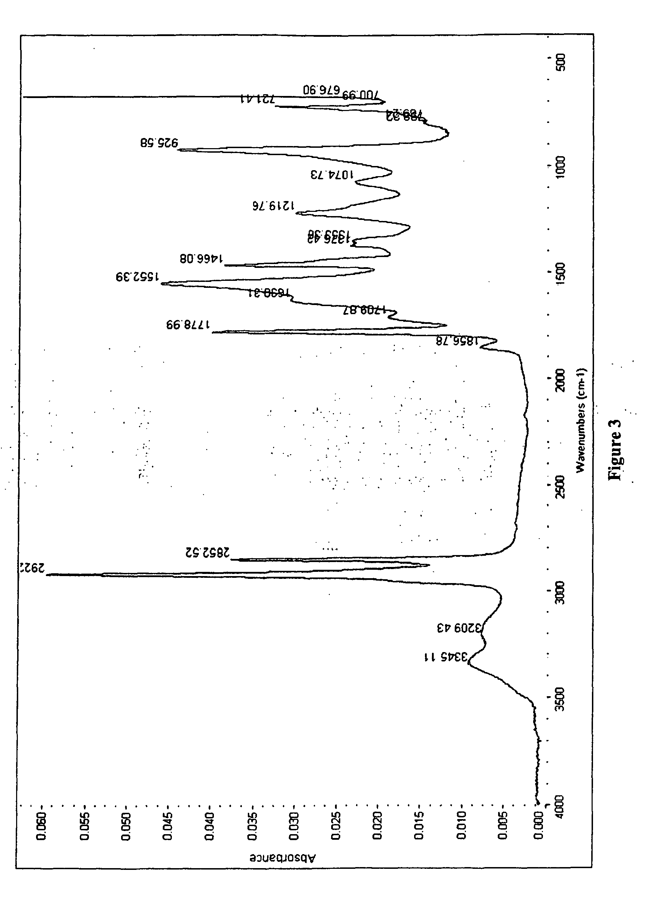

Figure 3 shows the IR spectrum of the PHMB treated with surface modified substrate. There is clearly a reduction in the strength of the anhydride signal.

This data shows the reaction of the PHMB with the surface modified polymer.

### XPS Data

X-ray Photoelectron Spectroscopy (XPS) is a surface sensitive technique that gives a specific signal for each element in the surface of a sample. As the binding energies of the atoms are altered slightly by the chemical environment of the atom, this data can also provide chemical bonding information.

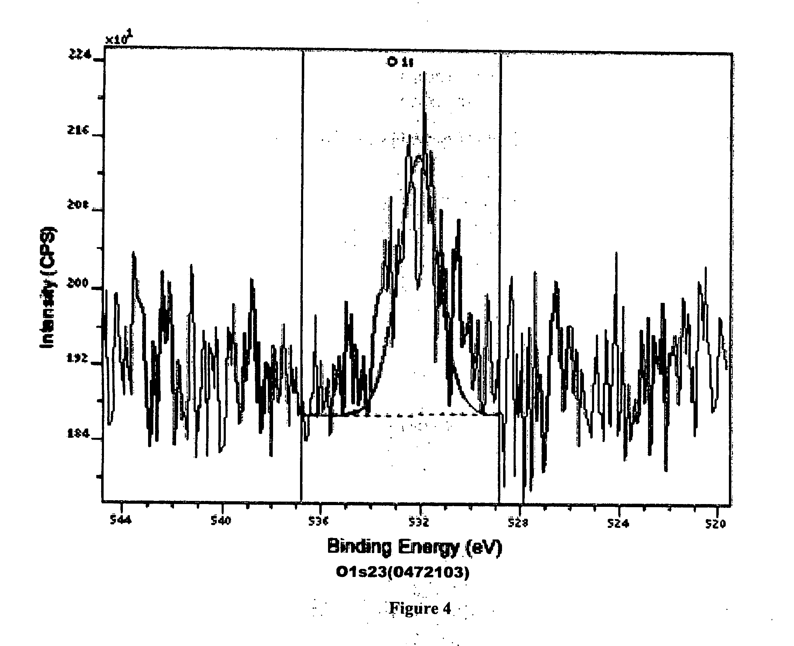

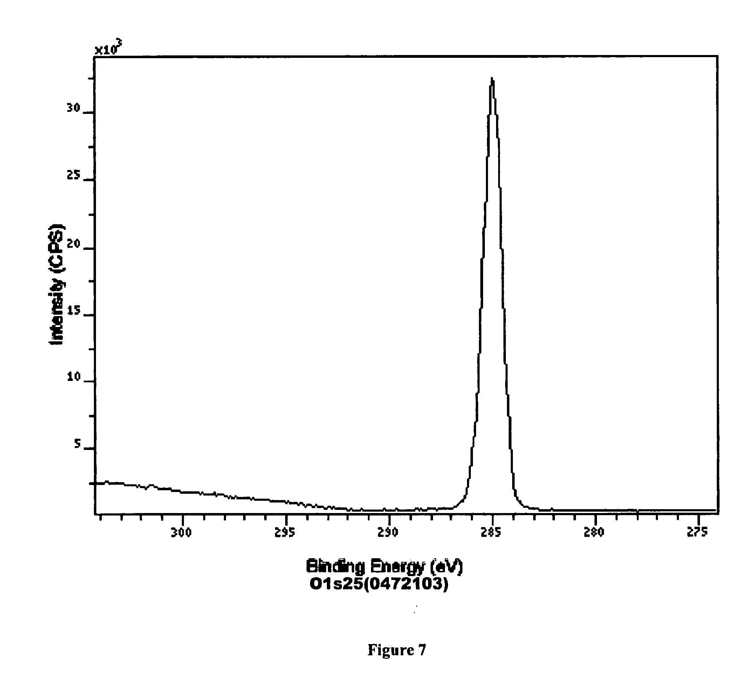

XPS was undertaken to look specifically at the outer surface of the material and determine which elements were present and how they are connected to the polymer surface. Figures 4-6 show O *1s* XPS data for polypropylene, PA-18 and PHMB modified PA-18. Figures 7-9 show C *1s* XPS data for polypropylene, PA-18, and PHMB modified PA-18.

### O 1s data

Figure 4 shows the *O1s* data for the unmodified polypropylene control. The only thing evident is a very weak C-O signal.

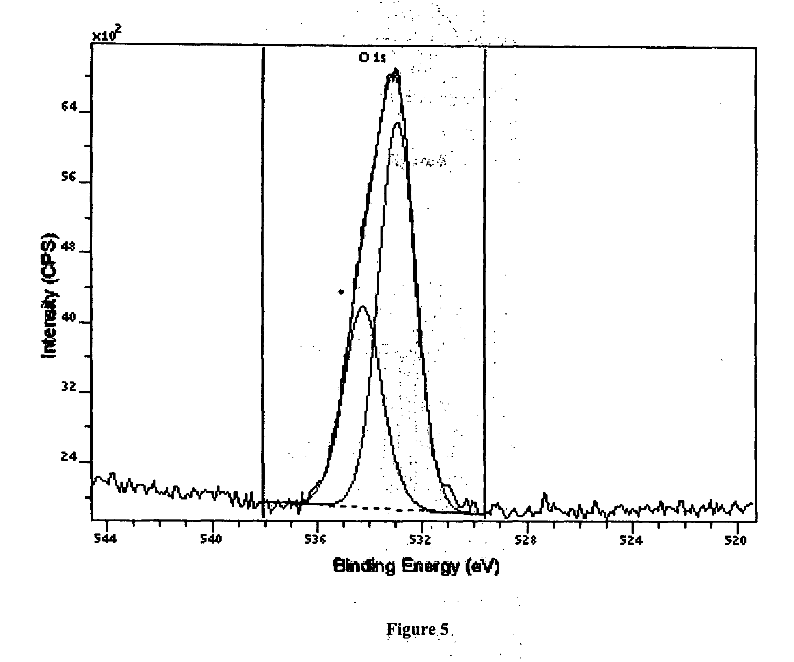

Figure 5 shows the O*1s* spectra for the PA-18 modified substrate. The spectra has two distinct O*1s* peaks at 533 and 534 eV. These two peaks are *O=C-O and O=C-O* and represent the anhydride functionality of the PA-18.

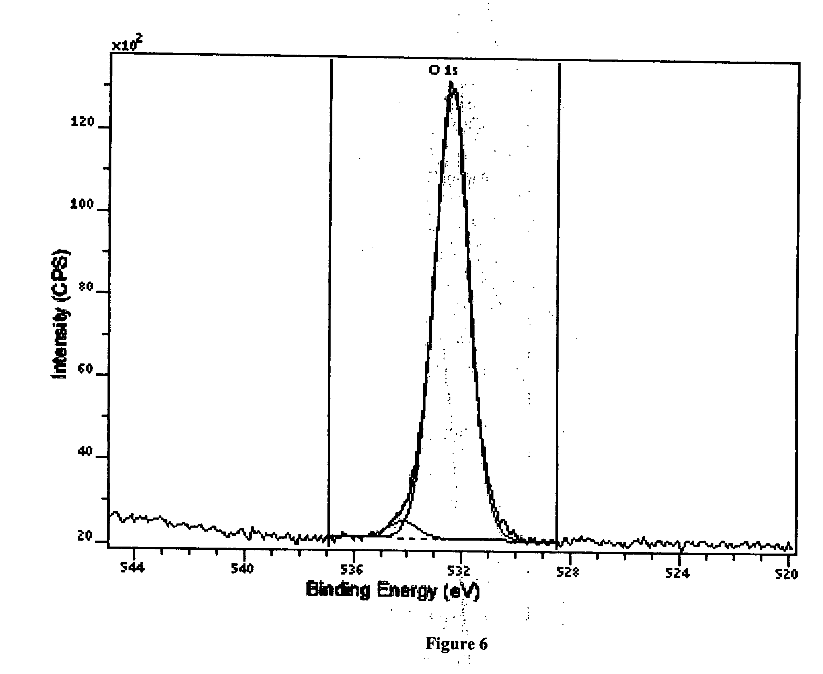

Figure 6 shows the O*1s* spectra for the PHMB treated PA-18. There is a new major peak at 532 eV which is due to the formation of the amide.

### C1s XPS data

Figure 7 shows the C *1s* spectra for pure polypropylene. This shows a single peak with a binding energy of 285 eV which corresponds to the aliphatic nature of the carbon in polypropylene.

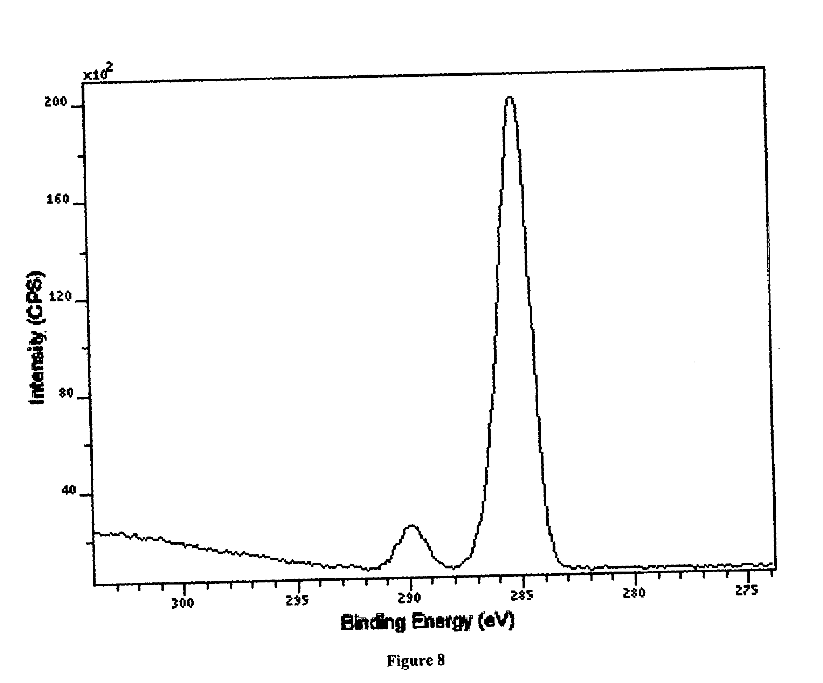

Figure 8 is the C *1s* spectra of a PA-18 film showing two distinct peaks at binding energy 285 and 290 eV. Again, the 285 eV is indicative of the aliphatic carbons present in the polymer while the new peak at 290 eV can be attributed to an ester bond which is indicative of the anhydride functionality.

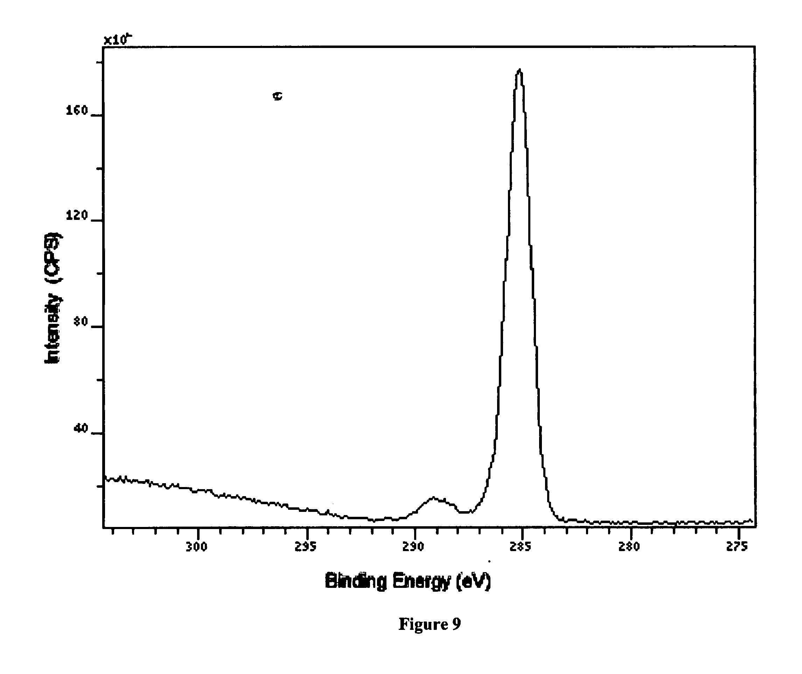

Figure 9 shows the C *1*s spectra for the PHMB modified PA-18. It also contains two peaks at 285 eV and one at 289 eV. The 289 eV is indicative of an amide peak (N-C=O) and is the product of the reaction between the anhydride of the PA-18 and an amine in the PHMB.

The XPS data clearly shows that the surface chemistry has been altered and that PHMB is covalently bound to the surface modified polypropylene surface.

### Microbiological Testing

To test the efficacy of the antimicrobial properties of the PHMB bound material Example 3, a simple test against E. *coli* was carried out. The performance of the PHMB modified substrate was compared with normal unmodified material and a control.

For each fibre sample, 0.3 grams of the fibre was immersed in 1 mL of water containing ~10⁶ E. *coli*/ mL. (Organisms per mL)

Aliquots from the supernatant of each sample were removed after 1 and 21 hours. A Colilert assay was used to determine the concentration of organisms in each sample. The results are displayed in Table 2.

**Table 2**

| Sample | *E. coli* concentration at 1 h Organisms per mL | *E. coli* concentration at 21 h Organisms per mL |
|---|---|---|
| Control | 1.3x10⁶ | 2.6x10⁵ |
| Polypropylene | 5.0x10⁵ | 1.3x10⁶ |
| Example 3 fibre ( PHMB) | <20 LOD (Limit of Detection - twenty organisms per mL) | <20 LOD |

The results show that the surface modified fibres with PHMB are highly effective against *E*. *coli.* After only an hour, essentially all of the *E*. *coli* was removed and the sample remained free of the microorganism after a further twenty hours.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A surface modified polyolefin with a non-leaching, reactive chemical functionality;
wherein the surface modified polyolefin comprises:
i) from 5% to 95% of a polyolefin; and
ii) from 5% to 95% of an amphiphilic polymer comprising reactive chemical functionality,
wherein at least a portion of the reactive chemical functionality is available for chemical reaction at the polyolefin surface.

2. The surface modified polyolefin of Claim 1 wherein the surface modified polyolefin comprises:
i) from 50% to 80% of a polyolefin; and
ii) from 20% to 50% of an amphiphilic polymer comprising reactive chemical functionality

3. The surface modified polyolefin of Claim 2 wherein the material comprises:
i) from 65% to 75% of a polyolefin; and
ii) from 25% to 35% of an amphiphilic polymer comprising reactive chemical functionality

4. The surface modified polyolefin according to any of the preceding claims wherein the polyolefin comprises polypropylene.

5. The surface modified polyolefin according to any of the preceding claims wherein the amphiphilic polymer comprises a polyanhydride.

6. The surface modified polyolefin of claim 5 wherein the polyanhydride comprises poly(maleic anhydride-*alt*-1-octadecene).

7. The surface modified polyolefin according to any of the preceding claims wherein an chemical entitity is covalently bound to the surface modified polyolefin.

8. The surface modified polyolefin according to claim 7 wherein the chemical entity is polyhexamethylene biguanide.

9. A fibre or film comprising at least one layer of the surface modified polyolefin from any of the preceding claims.

10. The fibre or film of claim 9 wherein the surface modified polyolefin constitutes between 5 % and 60 % of the total weight of the film or fibre.

11. A blow or injection molded item comprising at least one layer of the surface modified polyolefin according to any of claims 1 to 8.

12. A non-woven web suitable for use in an absorbent article wherein the web contains between 5 and 100 % by total weight of the surface modified polyolefin fibre according to any of claims 9 to 11.

13. A non-woven web according to claim 12 wherein the web contains between 20 % and 80 % of the surface modified polyolefin fibre.

14. A wet wipe, comprising the non woven substrate of claims 12 or 13, impregnated with a lotion.

15. The method of making the surface modified polyolefin of any of claims 1 to 8 which comprises hot melt extruding a mixture of the polyolefin and the amphiphilic polymer.
